# EUROPÄISCHE PATENTANMELDUNG

(11) **EP 1 295 877 A1**
(43) Veröffentlichungstag der Anmeldung: **26.03.2003**
(21) Anmeldenummer: 02020207.3
(22) Anmeldetag: 10.09.2002
(51) Int. Cl.: C07D 311/86, A01N 43/16

(54) **Xanthonderivate, Verfahren zu ihrer Herstellung und ihre Verwendung zur Bekämpfung von Schadpilzen sowie sie enthaltende Mittel**

(30) Priorität: 21.09.2001 DE 10146706
(71) Anmelder: BASF AKTIENGESELLSCHAFT, 67056 Ludwigshafen (DE)
(72) Erfinder: Rose, Ingo, Dr., 68159 Mannheim (DE); Tormo i Blasco, Jordi, Dr., 67117 Limburgerhof (DE); Gewehr, Markus, Dr., 56288 Kastellaun (DE); Grammenos, Wassilios, Dr., 67071 Ludwigshafen (DE); Müller, Bernd, Dr., 67227 Frankenthal (DE); Rheinheimer, Joachim, Dr., 67063 Ludwigshafen (DE); Schäfer, Peter, Dr., 67308 Ottersheim (DE); Schieweck, Frank, Dr., 67258 Hessheim (DE); Grote, Thomas, Dr., 67157 Wachenheim (DE); Gypser, Andreas, Dr., 68159 Mannheim (DE); Ammermann, Eberhard, Dr., 64646 Heppenheim (DE); Lorenz, Gisela, Dr., 67434 Hambach (DE); Stierl, Reinhard, Dr., 67112 Mutterstadt (DE); Strathmann, Siegfried, Dr., 67117 Limburgerhof (DE)

(57) **Zusammenfassung**

Xanthonderivate der Formel I 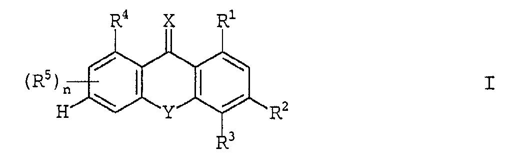 in der der Index und die Variablen folgende Bedeutung haben
- n: 0, 1 oder 2;
- R¹: Alkyl oder Halogenalkyl;
- R²,R³: Alkoxy, Alkenyloxy oder Alkinyloxy,
oder R² und R³ bilden gemeinsam eine ggf. subst. Oxy-alkylenoxygruppe;
- R⁴: Halogen, Cyano, Hydroxy, Amino, Mercapto, Alkyl, Halogenalkyl, Alkoxy, Halogenalkoxy, Alkylthio, Halogenalkylthio, Alkylcarbonyloxy oder Alkylcarbonylthio;
- R⁵: eine Gruppe R⁴, wobei die Gruppen R⁵ verschieden sein können, wenn n=2 ist;
- X, Y: Sauerstoff oder Schwefel;

Verfahren zur Herstellung dieser Verbindungen, sie enthaltende Mittel sowie ihre Verwendung zur Bekämpfung von pflanzenpathogenen Schadpilzen.

## Beschreibung

Die vorliegende Erfindung betrifft Xanthonderivate der Formel I 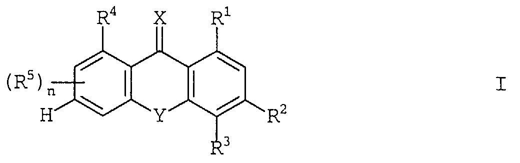 in der der Index und die Variablen folgende Bedeutung haben
- n: 0, 1 oder 2;
- R¹: C₁-C₄-Alkyl oder C₁-C₄-Halogenalkyl;
- R²,R³: unabhängig voneinander Wasserstoff, C₁-C₆-Alkoxy, C₃-C₆-Alkenyloxy oder C₃-C₆-Alkinyloxy,
oder R² und R³ bilden gemeinsam eine Oxy-C₁-C₄-Alkylenoxygruppe, die unsubstituiert oder durch 1 bis 4 der folgenden Reste substituiert ist: Halogen, Cyano, Hydroxy oder C₁-C₄-Alkyl;
- R⁴: Halogen, Cyano, Hydroxy, Amino, Mercapto, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylcarbonyloxy oder C₁-C₄-Alkylcarbonylthio;
- R⁵: eine Gruppe R⁴, wobei die Gruppen R⁵ verschieden sein können, wenn n=2 ist;
- X, Y: unabhängig voneinander Sauerstoff oder Schwefel.

Außerdem betrifft die Erfindung Verfahren zur Herstellung dieser Verbindungen, sie enthaltende Mittel sowie ihre Verwendung zur Bekämpfung von Schadpilzen.

Aus DE-A 4301424 sind Azaxanthone mit herbizider Wirkung bekannt.

Eine Wirkung von Xanthonderivaten gegen pflanzenpathogene Pilze ist im Stand der Technik bisher nicht beschrieben.

Der Erfindung lag als Aufgabe zugrunde, neue Verbindungen mit hoher Wirksamkeit gegen pflanzenpathogene Schadpilze zu finden.

Demgemäß wurden die eingangs definierten Verbindungen gefunden. Desweiteren wurden Verfahren zu ihrer Herstellung, sie enthaltende Mittel sowie deren Verwendung, bzw. Verfahren zur Bekämpfung von Schadpilzen unter Verwendung der Verbindungen I gefunden.

Die Verbindungen I können auf verschiedenen Wegen erhalten werden. Vorteilhaft geht man von Alkylbenzolderivaten der Formel II aus, in der R für eine C₁-C₄-Alkylgruppe steht, und Salicylsäurederivaten der Formel III, in der Q eine Schutzgruppe, insbesondere eine alkalisch abspaltbare Schutzgruppe, wie beispielsweise Wasserstoff oder Alkylcarbonyl, und Z Halogen, Alkoxy oder Hydroxy darstellt [vgl. T. W. Greene, Protective Groups in Organic Chemistry, J. Wiley & Sons, 1991, S. 10 - 142].

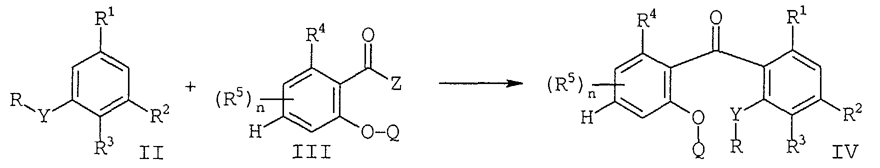

Die Acylierung von II zu IV erfolgt üblicherweise bei Temperaturen von -78°C bis 150°C, vorzugsweise 0°C bis 100°C, in einem inerten organischen Lösungsmittel in Gegenwart einer anorganischen oder organischen Säure, einer Lewis-Säure oder eines wasserentziehenden Mittels, wie beispielsweise P₂O₅ oder POCl₃ [vgl. Organikum, 20. Auflage, S. 359-363, Joh. A. Barth Verlag, Heidelberg und Leipzig (1996)].

Geeignete Lösungsmittel sind aliphatische Kohlenwasserstoffe, aromatische Kohlenwasserstoffe wie Nitrobenzol, halogenierte Kohlenwasserstoffe wie Methylenchlorid, Chloroform und Chlorbenzol, Ether, Nitrile, Ketone, sowie Dimethylsulfoxid, Dimethylformamid und Dimethylacetamid. Bevorzugt ist Nitrobenzol und halogenierte Kohlenwasserstoffe, besonders bevorzugt Dichlormethan. Es können auch Gemische der genannten Lösungsmittel verwendet werden.

Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Propionsäure, Oxalsäure, Toluolsulfonsäure, Benzolsulfonsäure, Camphersulfonsäure, Zitronensäure, Trifluormethansulfonsäure und Trifluoressigsäure, anorganische Säureanhydride wie Phosphorpentoxid, Polyphosphorsäure sowie Phosphoroxychlorid Verwendung.

Die Säuren werden im allgemeinen in katalytischen Mengen eingesetzt, sie können aber auch bis zu äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, II in einem Überschuß bezogen auf III einzusetzen.

Die Ausgangsstoffe II sind in der Literatur bekannt [Liebigs Ann. Chem., S. 220 (1969); JACS, Bd. 113, S. 8516-8518 (1991)] oder können gemäß der zitierten Literatur hergestellt werden.

Die Einführung der Schutzgruppe Q in die Salicylsäurederivate III erfolgt gemäß literaturbekannter Methoden [vgl. T. W. Greene, Protective Groups in Organic Chemistry, J. Wiley & Sons, 1991, S. 10 - 142]. Als Schutzgruppen kommen allgemein unter alkalischen Bedingungen abspaltbare Gruppen, wie C₁-C₆-Alkylcarbonyl oder Wasserstoff, insbesondere die Pivaloylgruppe in Betracht.

Die Cyclisierung von Verbindungen IV zu den Xanthonen der Formel I, in der X Sauerstoff bedeutet (Formel Ia), erfolgt allgemein bei Temperaturen von -78°C bis 150°C, vorzugsweise 0°C bis 50°C, in einem inerten Lösungsmittel in Gegenwart einer Base [vgl. Org. Prep. Proced. Int., Bd. 10, S. 79 (1978)].

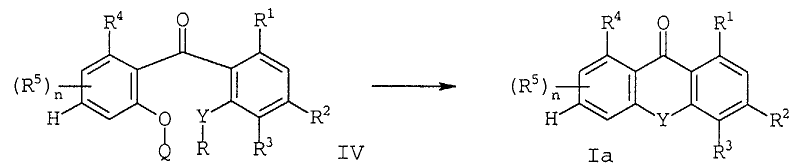

Geeignete Lösungsmittel sind Wasser, aliphatische Kohlenwasserstoffe, aromatische Kohlenwasserstoffe, halogenierte Kohlenwasserstoffe, Ether, Nitrile, Ketone, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt C₁-C₅-Alkohol-Wasser-Gemische. Es können auch Gemische der genannten Lösungsmittel verwendet werden.

Als Basen kommen allgemein anorganische Verbindungen wie Alkalimetall- und Erdalkalimetallhydroxide, Alkalimetall- und Erdalkalimetalloxide, Alkalimetall- und Erdalkalimetallhydride wie Lithiumhydrid, Natriumhydrid, Kaliumhydrid und Calziumhydrid, Alkalimetallamide, Alkalimetall- und Erdalkalimetallcarbonate wie Lithiumcarbonat, Kaliumcarbonat und Calziumcarbonat sowie Alkalimetallhydrogencarbonate wie Natriumhydrogencarbonat, metallorganische Verbindungen, insbesondere Alkalimetallalkyle, Alkylmagnesiumhalogenide sowie Alkalimetall- und Erdalkalimetallalkoholate und Dimethoxymagnesium, außerdem organische Basen, z.B. tertiäre Amine, Pyridin, substituierte Pyridine sowie bicyclische Amine in Betracht. Bevorzugt werden Alkalimetallhydroxide, Alkalimetallcarbonate, Alkalimetallhydrogencarbonate und Alkalimetallhydride. Besonders bevorzugt werden NaOH, KOH, NaHCO₃ und Na₂CO₃.

Die Abspaltung der Schutzgruppe aus IV und die Cyclisierung kann auch zweistufig erfolgen. Die Abspaltung der Schutzgruppe erfolgt bevorzugt bei Temperaturen von -50°C bis 100°C, vorzugsweise 0°C bis 50°C, in einem inerten Lösungsmittel, bevorzugt in Alkohol-Wasser-Gemischen in Gegenwart einer Base.

Als Basen kommen allgemein anorganische Verbindungen wie Alkalimetall- und Erdalkalimetallcarbonate wie Lithiumcarbonat, Kaliumcarbonat und Calziumcarbonat sowie Alkalimetallhydrogencarbonate wie Natriumhydrogencarbonat, in Betracht. Besonders bevorzugt wird Natriumhydrogencarbonat.

Die Cyclisierung der Verbindungen IV, in denen Q Wasserstoff bedeutet, erfolgt üblicherweise bei Temperaturen von 0°C bis 150°C, vorzugsweise 50°C bis 150°C, in einem inerten organischen Lösungsmittel in Gegenwart einer Base [vgl. Org. Prep. Proceed. Int. Bd. 10, S. 79 (1978)].

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie N-Methylpyrrolidon (NMP), Dimethylsulfoxid (DMSO), Dimethylformamid (DMF) und Dimethylacetamid (DMA), besonders bevorzugt Methanol, Ethanol DMSO und NMP. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide, Alkalimetall- und Erdalkalimetallalkoholate, Alkalimetall- und Erdalkalimetallcarbonate sowie Alkalimetallhydrogencarbonate wie Natriumhydrogencarbonat, in Betracht. Besonders bevorzugt wird Kaliumhydroxid.

Die Basen werden im allgemeinen äquimolar oder im Überschuß verwendet.

Xanthone I, in denen X und Y Sauerstoff bedeuten (Formel IA), sind auch ausgehend von o-Halogenbenzoesäurederivaten IIIa auf folgendem Weg zugänglich:

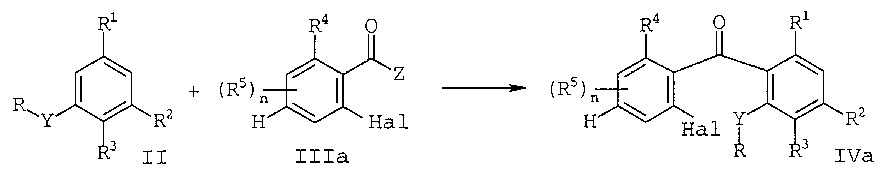

Die Verbindungen IVa sind aus Alkylbenzolderivaten der Formel II und Benzoesäurederivaten der Formel IIIa, in der Hal für Halogen, und insbesondere für Chlor steht und die anderen Variablen die Bedeutung wie in Formel III haben, unter den für die Acylierung von II mit III beschriebenen Bedingungen erhältlich. Bevorzugt wird die Acylierung von II mit IIIa in Gegenwart von P₂O₅ oder Polyphosphorsäure durchgeführt, wenn Z Hydroxy bedeutet. Wenn Z für Halogen steht, wird die Umsetzung in Gegenwart von AlCl₃ durchgeführt.

Falls Verbindungen I angestrebt werden, in denen R⁵ Halogen bedeutet, kann die Einführung der Gruppe R⁵ auf der Stufe der Verbindungen IVa erfolgen.

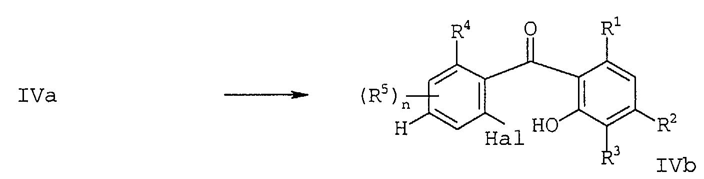

Die Umsetzung der Verbindungen IVa zu den Hydroxyverbindungen IVb erfolgt üblicherweise bei Temperaturen von -78°C bis 100°C, vorzugsweise 0°C bis 50°C, in einem inerten organischen Lösungsmittel in Gegenwart einer Lewissäure [vgl. T. W. Greene, Protective Groups in Organic Chemistry, J. Wiley & Sons, 1991, S.146 - 149].

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile, Ketone, Alkohole, sowie Dimethylsulfoxid, Dimethylformamid und Dimethylacetamid, besonders bevorzugt Tetrahydrofuran. Es können auch Gemische der genannten Lösungsmittel verwendet werden.

Als Lewissäuren kommen allgemein Haupt- und Nebengruppenhalogenide wie BF₃, AlCl₃, FeCl₃, SnCl₄, TiCl₄ oder ZnCl₂ in Betracht.

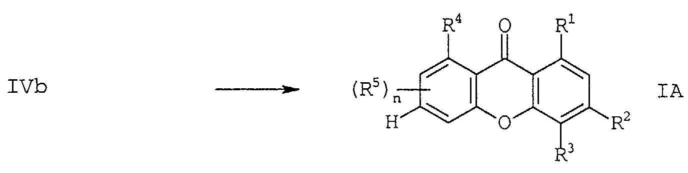

Die Cyclisierung von IVb zu den Verbindungen der Formel I, in der X und Y Sauerstoff bedeutet (Formel IA), erfolgt üblicherweise bei Temperaturen von -78°C bis 150°C, vorzugsweise 0°C bis 50°C, in einem inerten organischen Lösungsmittel in Gegenwart einer Base [vgl. Org. Prep. Proced. Int., Bd. 10, S. 79 (1978)].

Geeignete Lösungsmittel sind Wasser, aliphatische Kohlenwasserstoffe, aromatische Kohlenwasserstoffe, halogenierte Kohlenwasserstoffe, Ether, Nitrile, Ketone, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt C₁-C₅-Alkohol-Wasser-Gemische. Es können auch Gemische der genannten Lösungsmittel verwendet werden.

Als Basen kommen allgemein anorganische Verbindungen wie Alkalimetall- und Erdalkalimetallhydroxide, Alkalimetall- und Erdalkalimetalloxide, Alkalimetall- und Erdalkalimetallhydride wie Lithiumhydrid, Natriumhydrid, Kaliumhydrid und Calziumhydrid, Alkalimetallamide, Alkalimetall- und Erdalkalimetallcarbonate wie Lithiumcarbonat, Kaliumcarbonat und Calziumcarbonat sowie Alkalimetallhydrogencarbonate wie Natriumhydrogencarbonat, metallorganische Verbindungen, insbesondere Alkalimetallalkyle, Alkylmagnesiumhalogenide sowie Alkalimetall- und Erdalkalimetallalkoholate und Dimethoxymagnesium, außerdem organische Basen, z.B. tertiäre Amine, Pyridin, substituierte Pyridine sowie bicyclische Amine in Betracht. Bevorzugt werden Alkalimetallhydroxide, Alkalimetallcarbonate, Alkalimetallhydrogencarbonate und Alkalimetallhydride. Besonders bevorzugt werden NaOH, KOH, NaHCO₃ und Na₂CO₃.

Die Basen werden im allgemeinen in katalytischen Mengen eingesetzt, sie können aber auch äquimolar, im Überschuß oder gegebenenfalls als Lösungsmittel verwendet werden.

Ein alternativer Zugang zu Xanthonen der Formel IA, in der R⁴ Hydroxy, Halogen, C₁-C₄-Alkoxy oder C₁C₄-Halogenalkoxy bedeutet (Formel IA.1), besteht auch ausgehend von Alkylbenzolderivaten der Formel II.1 und 2,6-Dihalogen-Benzoesäurehalogeniden der Formel III.1. In Formel IA.1 haben die Variablen R¹ bis R³ und (R⁵)ₙ die Bedeutung wie in Formel I. In Formel II.1 haben R¹, R² und R³ die für Formel I gegebene Bedeutung und R^{x} steht für C₁-C₃-Alkoxy. In Formel III.1 steht Hal für Halogen, wie Chlor oder Brom, insbesondere für Chlor.

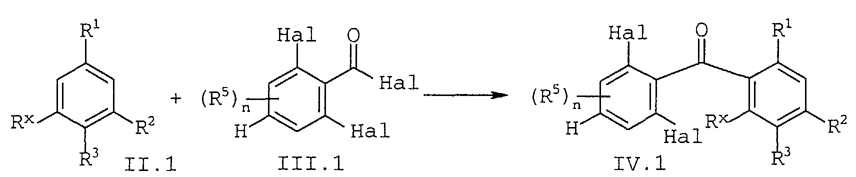

Diese Umsetzung erfolgt üblicherweise bei Temperaturen von 0°C bis 210°C, vorzugsweise 20°C bis 50°C, in einem inerten organischen Lösungsmittel in Gegenwart einer Lewissäure [vgl. Organikum, 20. Auflage, Kapitel 5, Joh. A. Barth Verlag, Heidelberg und Leipzig (1996) ].

Geeignete Lösungsmittel sind aliphatische Kohlenwasserstoffe, aromatische Kohlenwasserstoffe wie Toluol, Nitrobenzol, o-, mund p-Xylol, halogenierte Kohlenwasserstoffe, Ether, Nitrile, Ketone, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt Nitrobenzol oder halogenierte Kohlenwasserstoffe, insbesondere Methylenchlorid. Es können auch Gemische der genannten Lösungsmittel verwendet werden.

Als Lewissäuren kommen allgemein Haupt- und Nebengruppenhalogenide wie BF₃, AlCl₃, FeCl₃, SnCl₄, TiCl₄ oder ZnCl₂ in Betracht.

Falls R^{x} in Formel II.1 C₁-C₃-Alkoxy bedeutet, kann die Umsetzung zu IV.1 durch Wahl einer geeigneten Lewissäure, wie AlCl₃, direkt zu Hydroxyverbindungen IV.1, in denen R^{x} Hydroxy bedeutet, geführt werden.

Für Verbindungen IA.1, in denen R⁴ nicht Halogen bedeutet, erfolgt die Cyclisierung der Verbindungen IV.1 vorteilhaft unter gleichzeitiger Einführung der Gruppe R⁴.

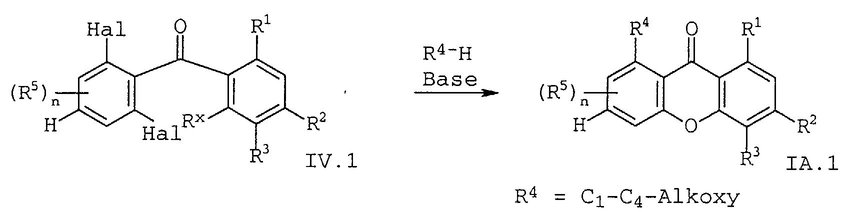

Diese Umsetzung erfolgt üblicherweise bei Temperaturen von -78°C bis 100°C, vorzugsweise 0°C bis 50°C, in einem inerten organischen Lösungsmittel in Gegenwart eines ggf. in situ hergestellten Alkali- oder Erdalkalimetallalkoholats.

Geeignete Lösungsmittel sind aliphatische oder aromatische Kohlenwasserstoffe, Ether, Nitrile, Ketone oder Alkohole wie Methanol, Ethanol, n-Propanol, Isopropanol, n-Butanol und tert.-Butanol, besonders bevorzugt Methanol. Es können auch Gemische der genannten Lösungsmittel verwendet werden.

Für die Alkali- oder Erdalkalialkoholatherstellung kommen insbesondere Natrium oder Kalium in Betracht.

Sie können äquimolar oder im Überschuß verwendet werden.

Verbindungen der Formel I, in denen R⁵ Halogen bedeutet und in 2-Stellung steht, werden bevorzugt aus entsprechend substituierten Salicylsäuren der Formel III hergestellt.

Verbindungen der Formel I, in denen R⁵ Halogen bedeutet und in 4-Stellung steht, werden bevorzugt durch Halogenierung aus den Xanthonen I hergestellt, in denen die betreffende Position unsubstituiert ist.

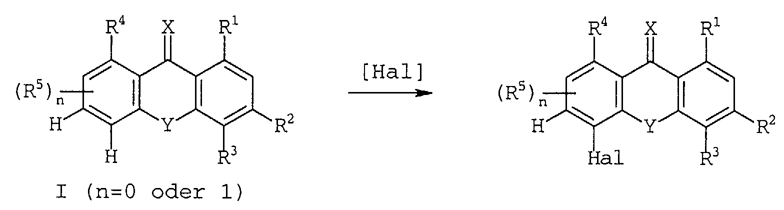

Die Halogenierung erfolgt unter allgemein literaturbekannten Bedingungen. Als Halogenierungsmittel kommen dabei insbesondere Brom oder Sulfurylchlorid in Betracht.

Ein Zugang zu Verbindungen der Formel IB besteht in der Schwefelung unter den aus GB-A 21 61 482 bekannten Bedingungen.

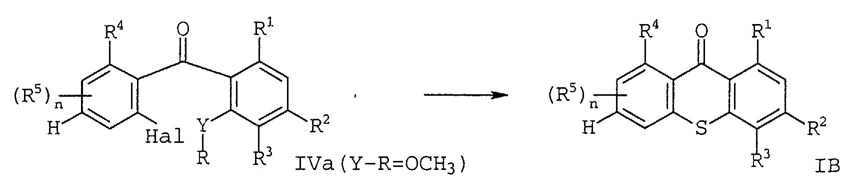

Als Schwefelungsagenz bei der Synthese der Verbindungen IB kommt insbesondere Natriumsulfid in Betracht.

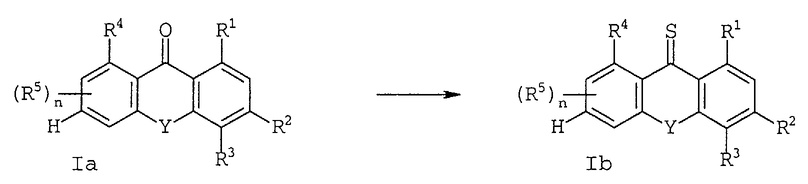

Zur Darstellung der Thioxanthone Ib erfolgt die Schwefelung von Ia unter literaturbekannten Bedingungen, sie erfolgt üblicherweise bei Temperaturen von 0°C bis 180°C, vorzugsweise 20°C bis 140°C, in einem inerten organischen Lösungsmittel [vgl. Liebigs Ann. Chem., S. 177 (1989)].

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, sowie Dimethylsulfoxid, besonders bevorzugt Toluol und Tetrahydrofuran. Es können auch Gemische der genannten Lösungsmittel verwendet werden.

Als Schwefelungsagentien kommen beispielsweise Phosphorpentasulfid oder das Lawesson-Reagenz in Betracht.

Die Edukte werden im allgemeinen in äquimolaren Mengen miteinander umgesetzt. Es kann für die Ausbeute vorteilhaft sein, das Schwefelungsreagenz in einem Überschuß bezogen auf Ia einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischenund Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Jod;
**Alkyl:** gesättigte, geradkettige oder verzweigte Kohlenwasserstoffreste mit 1 bis 4 oder 6 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Halogenalkyl:** geradkettige oder verzweigte Alkylgruppen mit 1 bis 4 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkoxy:** geradkettige oder verzweigte Alkylgruppen mit 1 bis 6 Kohlenstoffatomen (wie vorstehend genannt), welche über ein Sauerstoffatom (-O-) an das Gerüst gebunden sind;
**Halogenalkoxy:** geradkettige oder verzweigte Halogenalkylgruppen mit 1 bis 6 Kohlenstoffatomen (wie vorstehend genannt), welche über ein Sauerstoffatom (-O-) an das Gerüst gebunden sind;
**Alkylthio:** geradkettige oder verzweigte Alkylgruppen mit 1 bis 4 Kohlenstoffatomen (wie vorstehend genannt), welche über ein Schwefelatom (-S-) an das Gerüst gebunden sind;
**Alkylcarbonyl:** eine geradkettige oder verzweigte Alkylgruppe mit 1 bis 10 Kohlenstoffatomen (wie vorstehend genannt), welche über eine Carbonylgruppe (-CO-) an das Gerüst gebunden ist;
**Alkylcarbonyloxy:** eine geradkettige oder verzweigte Alkylcarbonylgruppe mit 1 bis 10 Kohlenstoffatomen (wie vorstehend genannt), welche über Sauerstoff an das Gerüst gebunden ist;
**Alkenyl:** ungesättigte, geradkettige oder verzweigte Kohlenwasserstoffreste mit 3 bis 4 oder 6 Kohlenstoffatomen und einer Doppelbindung in einer beliebigen Position, z.B. C₃-C₆-Alkenyl wie 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-penteny 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-l-butenyl, 1,2-Dimethyl-2-butenyl, 1,2-Dimethyl-3-butenyl, 1,3-Dimethyl-l-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1propenyl und 1-Ethyl-2-methyl-2-propenyl;
**Halogenalkenyl:** ungesättigte, geradkettige oder verzweigte Kohlenwasserstoffreste mit 3 bis 6 Kohlenstoffatomen und einer Doppelbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
**Alkenyloxy:** ungesättigte, geradkettige oder verzweigte Kohlenwasserstoffreste mit 3 bis 6 Kohlenstoffatomen und einer Doppelbindung in einer beliebigen, nicht zum Heteroatom benachbarten, Position (wie vorstehend genannt), welche über ein Sauerstoffatom (-O-) an das Gerüst gebunden sind;
**Halogenalkenyloxy:** ungesättigte, geradkettige oder verzweigte Alkenyloxygruppen mit 3 bis 6 Kohlenstoffatomen (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
**Alkinyl:** geradkettige oder verzweigte Kohlenwasserstoffgruppen mit 3 bis 4 oder 6 Kohlenstoffatomen und einer Dreifachbindung in einer beliebigen Position, z.B. C₃-C₆-Alkinyl wie 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentiny 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-l-butinyl, 1-Ethyl-2-butinyl, 1-Ethyl-3-butinyl, 2-Ethyl-3-butinyl und 1-Ethyl-l-methyl-2-propinyl;
**Halogenalkinyl:** ungesättigte, geradkettige oder verzweigte Kohlenwasserstoffreste mit 3 bis 6 Kohlenstoffatomen und einer Dreifachbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
**Alkinyloxy:** ungesättigte, geradkettige oder verzweigte Kohlenwasserstoffreste mit 3 bis 10 Kohlenstoffatomen und einer Dreifachbindung in einer beliebigen, nicht zum Heteroatom benachbarten, Position (wie vorstehend genannt), welche über ein Sauerstoffatom (-O-) an das Gerüst gebunden sind;
**Halogenalkinyloxy:** ungesättigte, geradkettige oder verzweigte Alkinyloxygruppen mit 3 bis 10 Kohlenstoffatomen (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können.
**Alkylen:** divalente unverzweigte Ketten aus 3 bis 5 CH₂-Gruppen, z.B. CH₂, CH₂CH₂, CH₂CH₂CH₂, CH₂CH₂CH₂CH₂ und CH₂CH₂CH₂CH₂CH₂;
**Oxyalkylen:** divalente unverzweigte Ketten aus 2 bis 4 CH₂-Gruppen, wobei eine Valenz über ein Sauerstoffatom an das Gerüst gebunden ist, z.B. OCH₂CH₂, OCH₂CH₂CH₂ und OCH₂CH₂CH₂CH₂;
**Oxyalkylenoxy:** divalente unverzweigte Ketten aus 1 bis 3 CH₂-Gruppen, wobei beide Valenzen über ein Sauerstoffatom an das Gerüst gebunden ist, z.B. OCH₂O, OCH₂CH₂O und OCH₂CH₂CH₂O;

Im Hinblick auf ihre bestimmungsgemäße Verwendung der Xanthonverbindungen der Formel I sind die folgenden Bedeutungen der Substituenten, und zwar jeweils für sich allein oder in Kombination, besonders bevorzugt:

Verbindungen I werden bevorzugt, in denen Y Sauerstoff bedeutet.

Daneben werden Verbindungen I bevorzugt, in denen X und Y Sauerstoff bedeuten. Sie entsprechen Formel I.1.

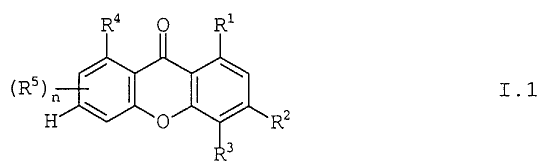

Außerdem werden Verbindungen I besonders bevorzugt, in denen R¹ für Methyl, Halogenmethyl oder Ethyl, insbesondere für Methyl steht.

Gleichermaßen bevorzugt sind Verbindungen I, in denen R² und R³ unabhängig voneinander Methoxy, Ethoxy oder n-Butoxy, insbesondere Methoxy oder n-Butoxy, besonders bevorzugt Methoxy bedeuten.

Außerdem werden Verbindungen I bevorzugt, in denen ein Rest R² oder R³ Methoxy bedeutet.

Insbesondere bevorzugt werden Verbindungen I, in denen R¹ Methyl und R² und R³ Methoxy bedeuten.

Weiterhin besonders bevorzugt werden Verbindungen I, in denen R⁴ Halogen, Cyano, Hydroxy, Methyl, Methoxy, Alkylcarbonyloxy, Halogenmethyl oder Halogenmethoxy bedeutet.

Daneben werden Verbindungen I besonders bevorzugt, in denen R⁴ für Fluor, Chlor, Brom, Jod, Hydroxy, Amino, Methyl, C₁-C₄-Halogenalkyl, Methoxy, C₁-C₄-Halogenalkoxy oder C₁-C₄-Alkylthio steht.

Gleichermaßen besonders bevorzugt sind Verbindungen I, in denen R⁴ für Hydroxy, Amino, Methyl, C₁-Halogenalkyl, Methoxy, C₁-Halogenalkoxy oder C₁-Alkylthio steht.

Verbindungen I werden bevorzugt, in denen R⁴ für OCHₘF₃₋ₘ, wobei m eine ganze Zahl von 0 bis 3 ist, insbesondere für OCF₃ oder OCHF₂ steht.

Daneben werden Verbindungen I besonders bevorzugt, in denen R⁵ für Halogen, insbesondere für Chlor oder Brom steht.

Besonders werden auch Verbindungen I bevorzugt, in denen n für 1 steht.

Weiterhin werden Verbindungen I bevorzugt, in denen R⁴ OCHₘF₃₋ₘ, und R⁵ Halogen oder OCHₘF₃₋ₘ, wobei m eine ganze Zahl von 0 bis 2 ist, bedeuten.

Außerdem werden Verbindungen I bevorzugt, in denen R¹ C₁-C₄-Alkyl oder CF₃, R² und R³ jeweils gleich oder verschieden sein können und C₁-C₄-Alkoxy bedeuten, R⁴ für Halogen, C₁-C₄-Alkyl, CF₃, CHF₂, Hydroxy, C₁-C₄-Alkoxy, OCHF₂, C₁-C₄-Alkoxycarbonyl, Mercapto, oder Amino und (R⁵)ₙ für Wasserstoff, Halogen, Methyl, Hydroxy, CF₃ oder OCHF₂ steht.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

### Tabelle 1

Verbindungen der Formel I.1, in denen R¹ für Methyl, R² und R³ für Methoxy stehen und die Kombination der Reste R⁴ und (R⁵)ₙ für eine Verbindung jeweils einer Zeile der Tabelle A entspricht

### Tabelle 2

Verbindungen der Formel I.1, in denen R¹ für Methyl, R² für Methoxy und R³ für Ethoxy stehen und die Kombination der Reste R⁴ und (R⁵)ₙ für eine Verbindung jeweils einer Zeile der Tabelle A entspricht

### Tabelle 3

Verbindungen der Formel I.1, in denen R¹ für Methyl, R² für Methoxy und R³ für n-Propoxy stehen und die Kombination der Reste R⁴ und (R⁵)ₙ für eine Verbindung jeweils einer Zeile der Tabelle A entspricht

### Tabelle 4

Verbindungen der Formel I.1, in denen R¹ für Methyl, R² für Methoxy und R³ für i-Propoxy stehen und die Kombination der Reste R⁴ und (R⁵)ₙ für eine Verbindung jeweils einer Zeile der Tabelle A entspricht

### Tabelle 5

Verbindungen der Formel I.1, in denen R¹ für Methyl, R² für Methoxy und R³ für n-Butoxy stehen und die Kombination der Reste R⁴ und (R⁵)ₙ für eine Verbindung jeweils einer Zeile der Tabelle A entspricht

### Tabelle 6

Verbindungen der Formel I.1, in denen R¹ für Methyl, R² für Ethoxy und R³ für Methoxy stehen und die Kombination der Reste R⁴ und (R⁵)ₙ für eine Verbindung jeweils einer Zeile der Tabelle A entspricht

### Tabelle 7

Verbindungen der Formel I.1, in denen R¹ für Methyl, R² für n-Propoxy und R³ für Methoxy stehen und die Kombination der Reste R⁴ und (R⁵)ₙ für eine Verbindung jeweils einer Zeile der Tabelle A entspricht

### Tabelle 8

Verbindungen der Formel I.1, in denen R¹ für Methyl, R² für i-Propoxy und R³ für Methoxy stehen und die Kombination der Reste R⁴ und (R⁵)ₙ für eine Verbindung jeweils einer Zeile der Tabelle A entspricht

### Tabelle 9

Verbindungen der Formel I.1, in denen R¹ für Methyl, R² für n-Butoxy und R³ für Methoxy stehen und die Kombination der Reste R⁴ und (R⁵)ₙ für eine Verbindung jeweils einer Zeile der Tabelle A entspricht

### Tabelle 10

Verbindungen der Formel I.1, in denen R¹ für Chlormethyl, R² und R³ für Methoxy stehen und die Kombination der Reste R⁴ und (R⁵)ₙ für eine Verbindung jeweils einer Zeile der Tabelle A entspricht

### Tabelle 11

Verbindungen der Formel I.1, in denen R¹ für Chlormethyl, R² für Methoxy und R³ für Ethoxy stehen und die Kombination der Reste R⁴ und (R⁵)ₙ für eine Verbindung jeweils einer Zeile der Tabelle A entspricht

### Tabelle 12

Verbindungen der Formel I.1, in denen R¹ für Chlormethyl, R² für Methoxy und R³ für n-Propoxy stehen und die Kombination der Reste R⁴ und (R⁵)ₙ für eine Verbindung jeweils einer Zeile der Tabelle A entspricht

### Tabelle 13

Verbindungen der Formel I.1, in denen R¹ für Chlormethyl, R² für Methoxy und R³ für i-Propoxy stehen und die Kombination der Reste R⁴ und (R⁵)ₙ für eine Verbindung jeweils einer Zeile der Tabelle A entspricht

### Tabelle 14

Verbindungen der Formel I.1, in denen R¹ für Chlormethyl, R² für Methoxy und R³ für n-Butoxy stehen und die Kombination der Reste R⁴ und (R⁵)ₙ für eine Verbindung jeweils einer Zeile der Tabelle A entspricht

### Tabelle 15

Verbindungen der Formel I.1, in denen R¹ für Chlormethyl, R² für Ethoxy und R³ für Methoxy stehen und die Kombination der Reste R⁴ und (R⁵)ₙ für eine Verbindung jeweils einer Zeile der Tabelle A entspricht

### Tabelle 16

Verbindungen der Formel I.1, in denen R¹ für Chlormethyl, R² und R³ für Ethoxy stehen und die Kombination der Reste R⁴ und (R⁵)ₙ für eine Verbindung jeweils einer Zeile der Tabelle A entspricht

### Tabelle 17

Verbindungen der Formel I.1, in denen R¹ für Chlormethyl, R² für n-Propoxy und R³ für Methoxy stehen und die Kombination der Reste R⁴ und (R⁵)ₙ für eine Verbindung jeweils einer Zeile der Tabelle A entspricht

### Tabelle 18

Verbindungen der Formel I.1, in denen R¹ für Chlormethyl, R² für i-Propoxy und R³ für Methoxy stehen und die Kombination der Reste R⁴ und (R⁵)ₙ für eine Verbindung jeweils einer Zeile der Tabelle A entspricht

### Tabelle 19

Verbindungen der Formel I.1, in denen R¹ für Chlormethyl, R² für n-Butoxy und R³ für Methoxy stehen und die Kombination der Reste R⁴ und (R⁵)ₙ für eine Verbindung jeweils einer Zeile der Tabelle A entspricht

### Tabelle 20

Verbindungen der Formel I.1, in denen R¹ für Fluormethyl, R² und R³ für Methoxy stehen und die Kombination der Reste R⁴ und (R⁵)ₙ für eine Verbindung jeweils einer Zeile der Tabelle A entspricht

### Tabelle 21

Verbindungen der Formel I.1, in denen R¹ für Fluormethyl, R² für Methoxy und R³ für Ethoxy stehen und die Kombination der Reste R⁴ und (R⁵)ₙ für eine Verbindung jeweils einer Zeile der Tabelle A entspricht

### Tabelle 22

Verbindungen der Formel I.1, in denen R¹ für Fluormethyl, R² für Methoxy und R³ für n-Propoxy stehen und die Kombination der Reste R⁴ und (R⁵)ₙ für eine Verbindung jeweils einer Zeile der Tabelle A entspricht

### Tabelle 23

Verbindungen der Formel I.1, in denen R¹ für Fluormethyl, R² für Methoxy und R³ für i-Propoxy stehen und die Kombination der Reste R⁴ und (R⁵)ₙ für eine Verbindung jeweils einer Zeile der Tabelle A entspricht

### Tabelle 24

Verbindungen der Formel I.1, in denen R¹ für Fluormethyl, R² für Methoxy und R³ für n-Butoxy stehen und die Kombination der Reste R⁴ und (R⁵)ₙ für eine Verbindung jeweils einer Zeile der Tabelle A entspricht

### Tabelle 25

Verbindungen der Formel I.1, in denen R¹ für Fluormethyl, R² für Ethoxy und R³ für Methoxy stehen und die Kombination der Reste R⁴ und (R⁵)ₙ für eine Verbindung jeweils einer Zeile der Tabelle A entspricht

### Tabelle 26

Verbindungen der Formel I.1, in denen R¹ für Fluormethyl, R² für n-Propoxy und R³ für Methoxy stehen und die Kombination der Reste R⁴ und (R⁵)ₙ für eine Verbindung jeweils einer Zeile der Tabelle A entspricht

### Tabelle 27

Verbindungen der Formel I.1, in denen R¹ für Fluormethyl, R² für i-Propoxy und R³ für Methoxy stehen und die Kombination der Reste R⁴ und (R⁵)ₙ für eine Verbindung jeweils einer Zeile der Tabelle A entspricht

### Tabelle 28

Verbindungen der Formel I.1, in denen R¹ für Fluormethyl, R² für n-Butoxy und R³ für Methoxy stehen und die Kombination der Reste R⁴ und (R⁵)ₙ für eine Verbindung jeweils einer Zeile der Tabelle A entspricht

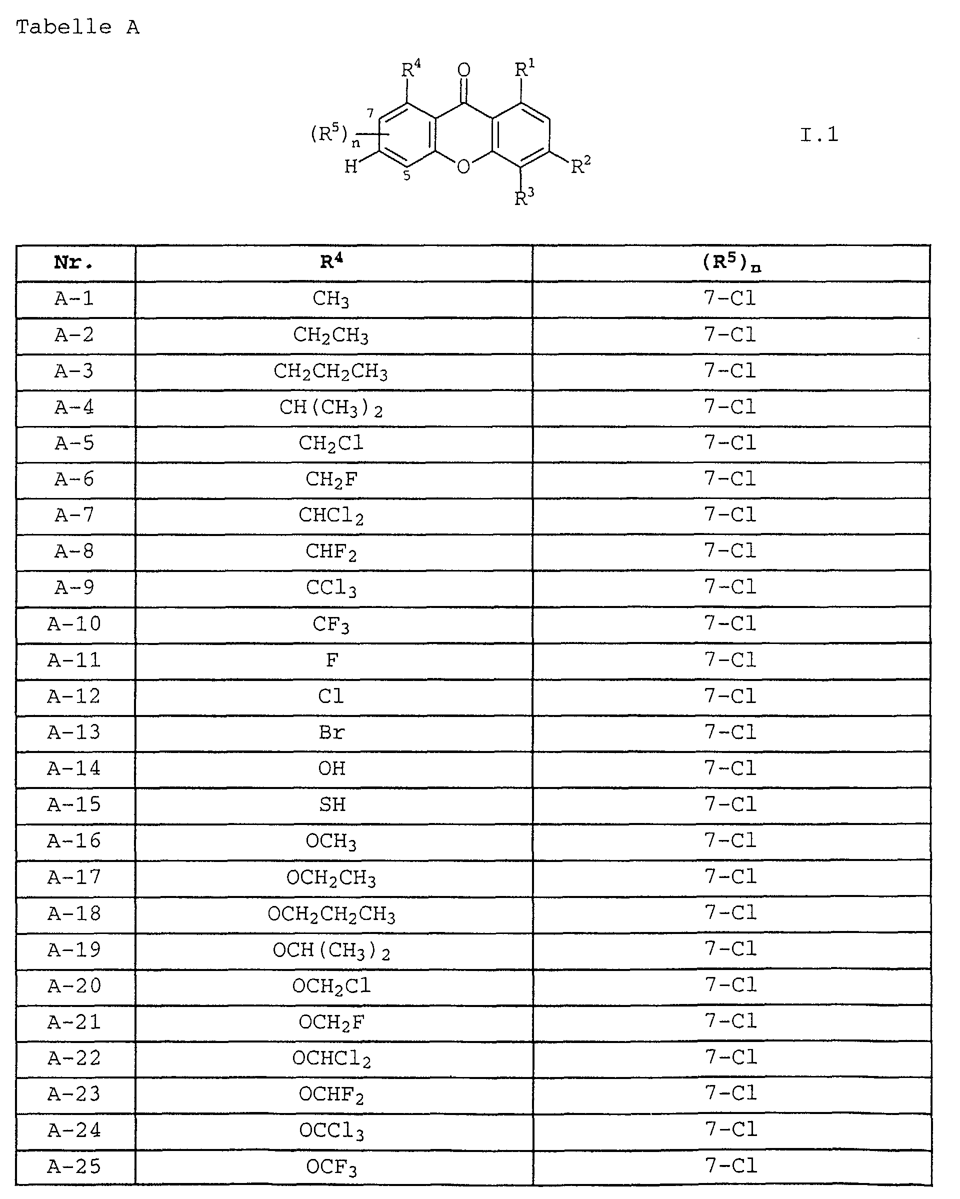

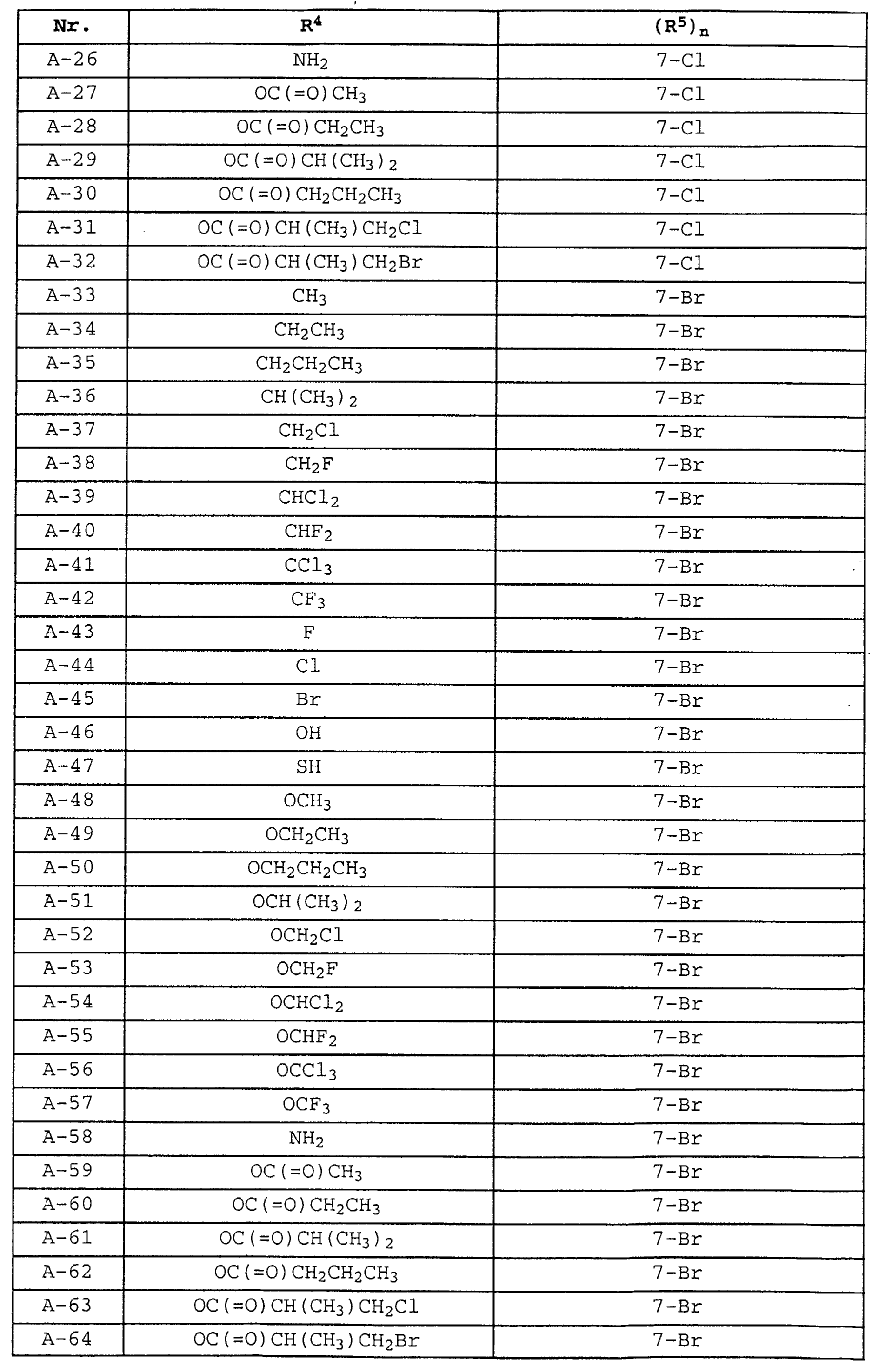

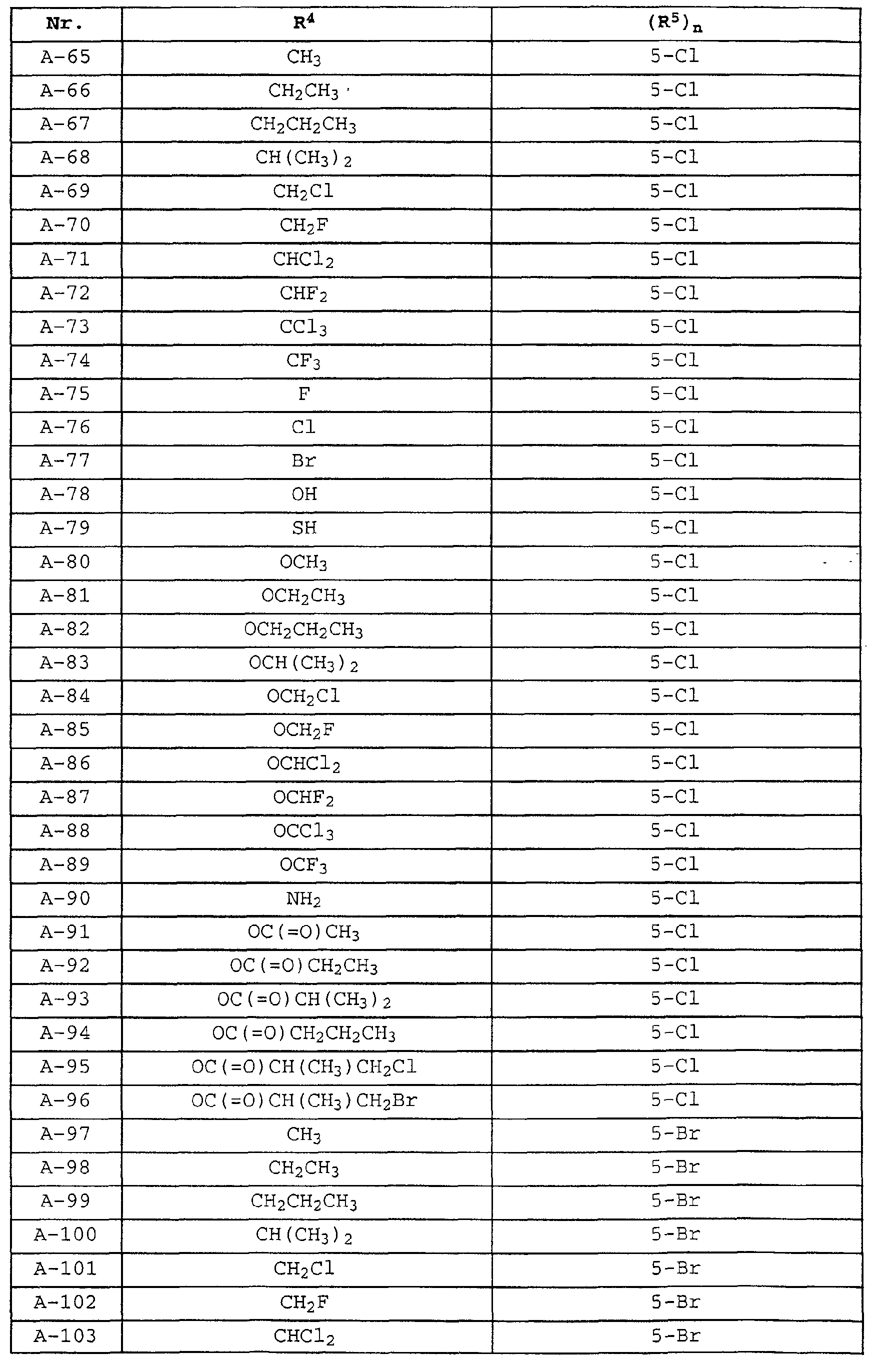

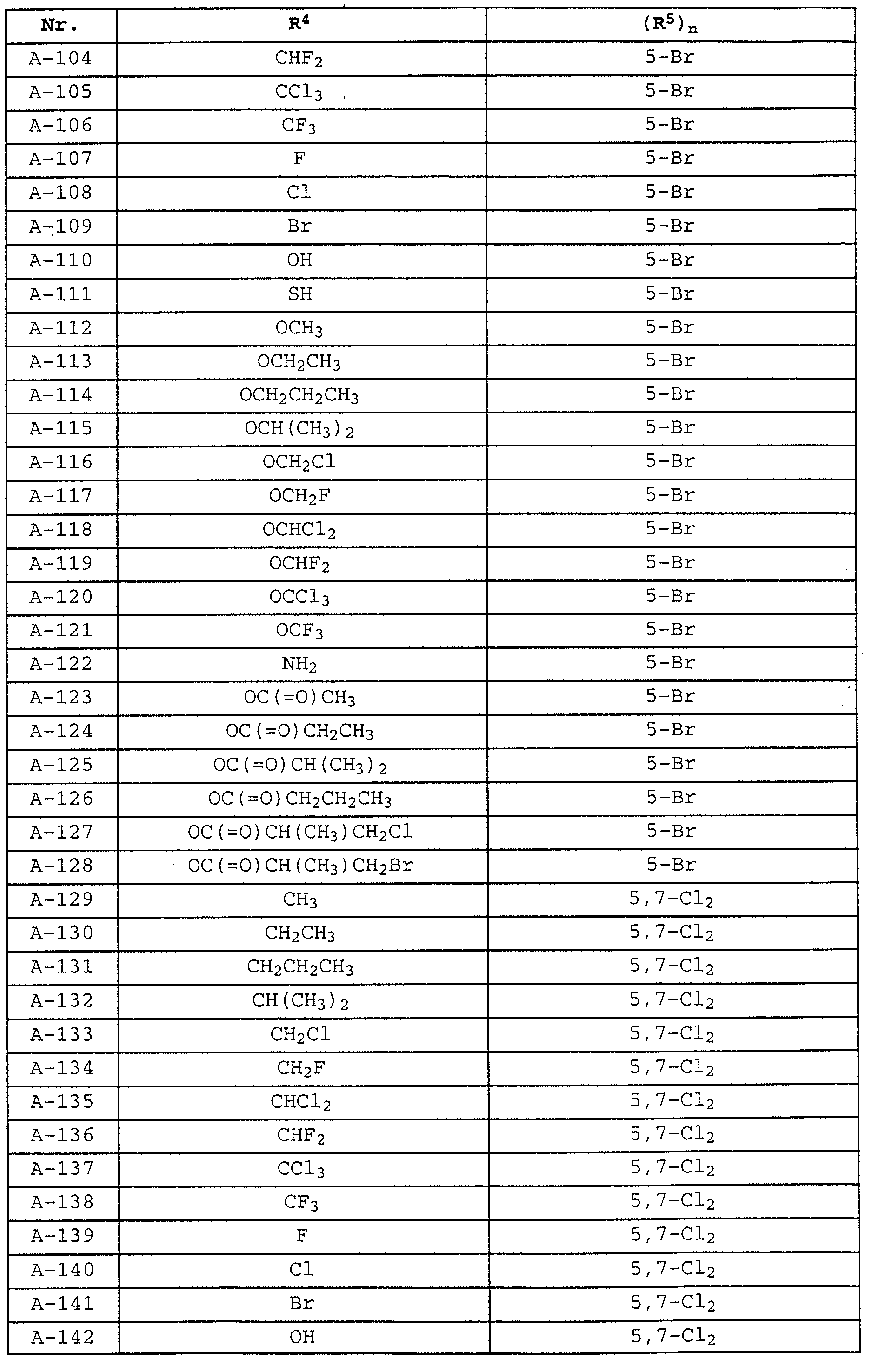

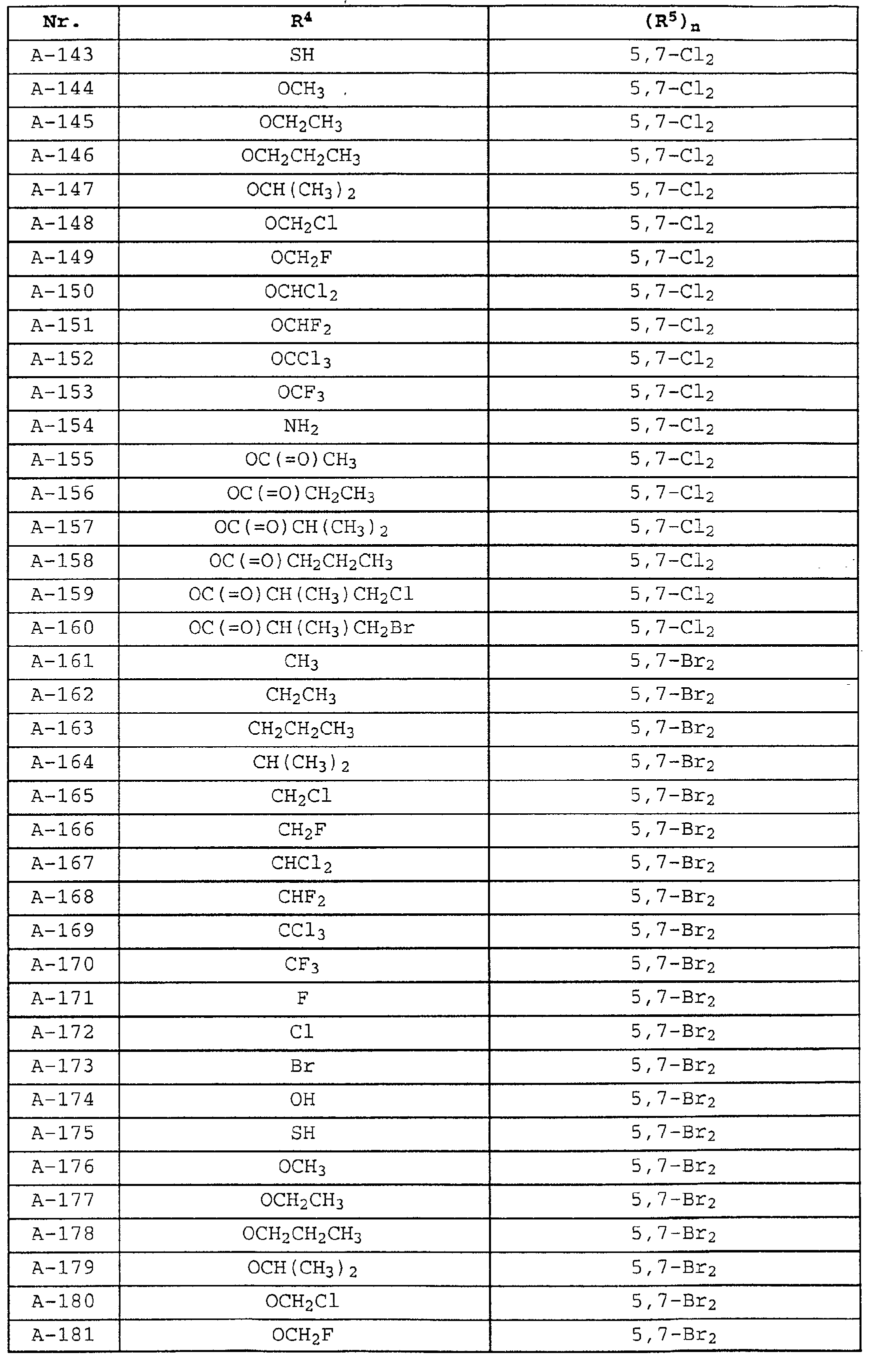

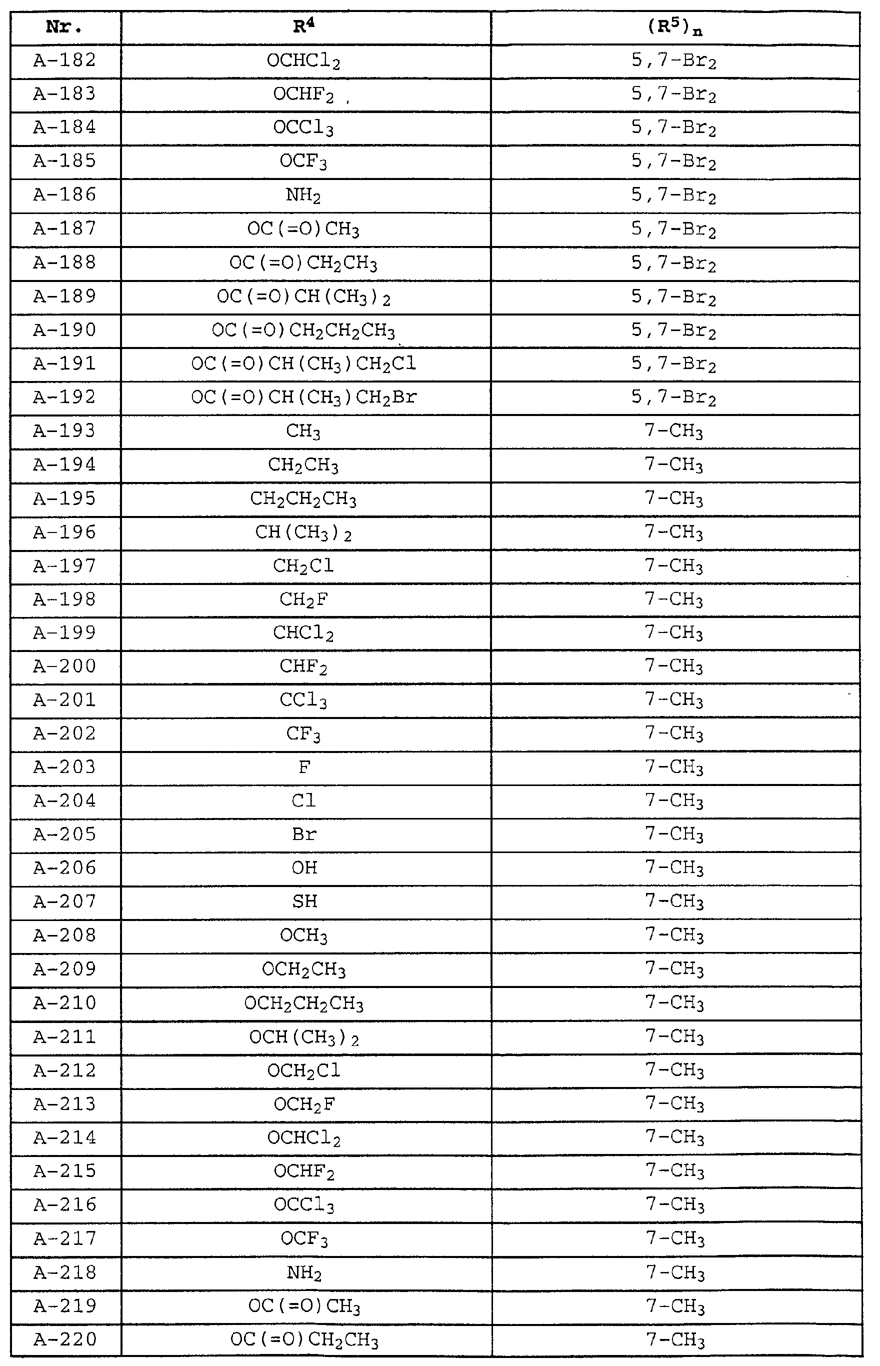

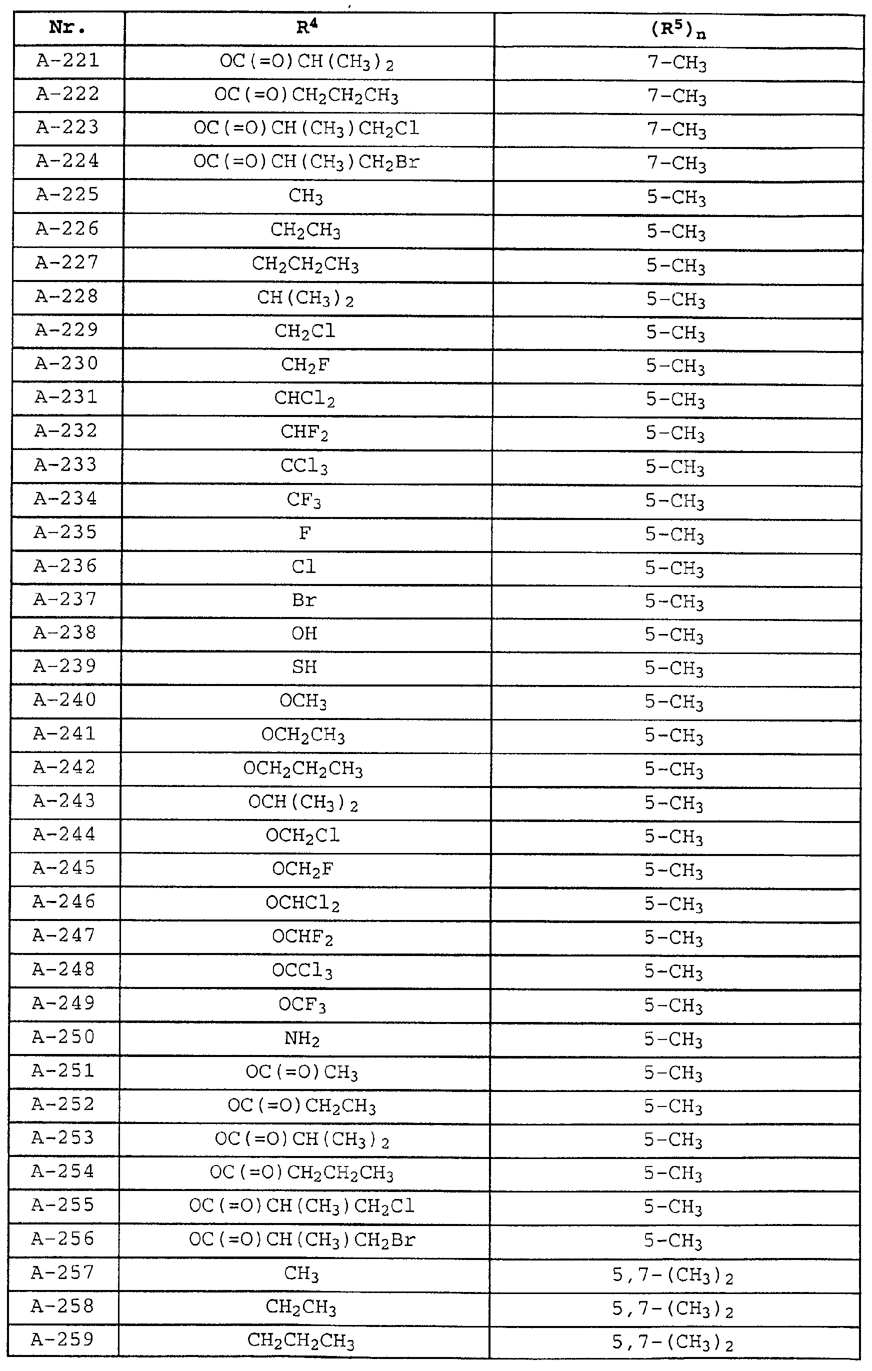

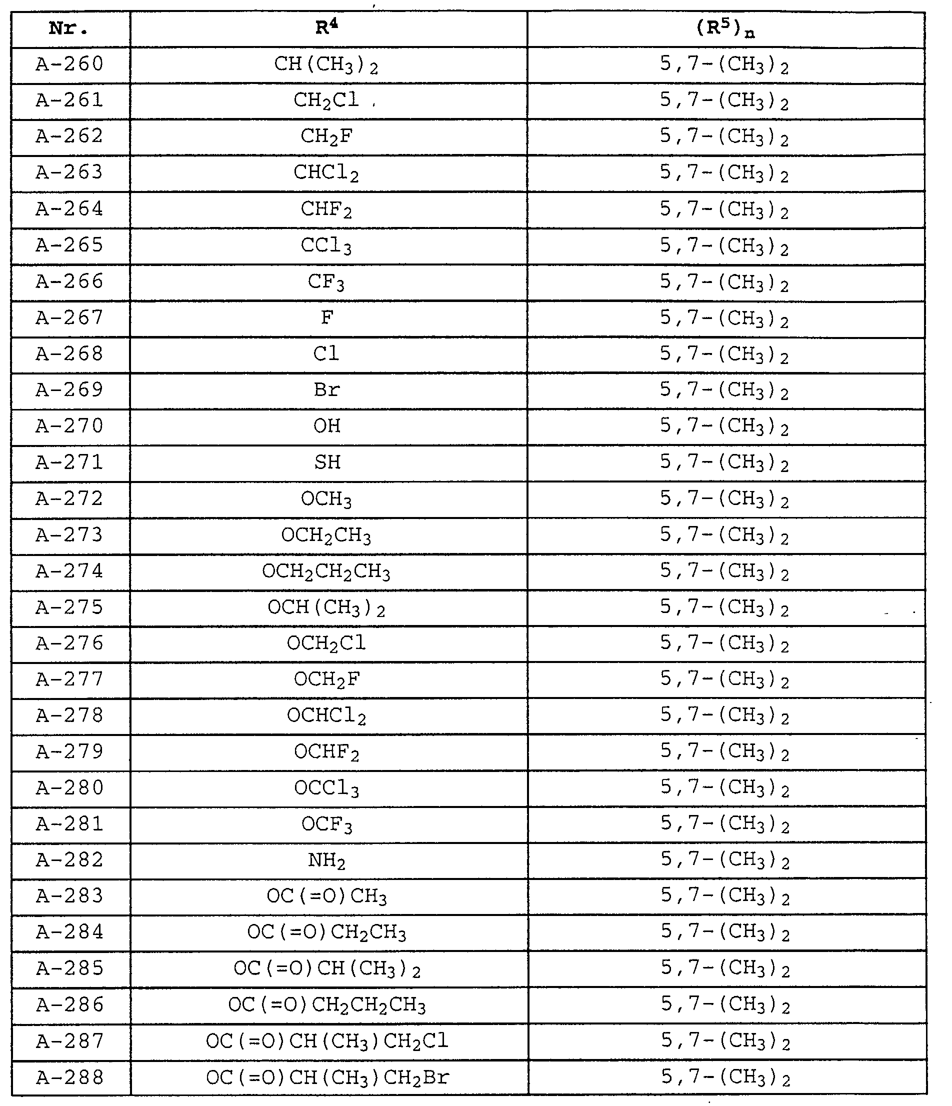

Die Verbindungen I eignen sich als Fungizide. Sie zeichnen sich durch eine hervorragende Wirksamkeit gegen ein breites Spektrum von pflanzenpathogenen Pilzen, insbesondere aus der Klasse der *Ascomyceten, Deuteromyceken, Phycomyceten* und *Basidiomyceten*, aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- Alternaria-Arten, Podosphaera-Arten, Sclerotinia-Arten, Physalospora canker an Gemüse und Obst,
- Botrytis cinerea (Grauschimmel) an Erdbeeren, Gemüse, Zierpflanzen und Reben,
- Corynespora cassiicola an Gurken,
- Colletotrichum-Arten an Obst und Gemüse,
- Diplocarpon rosae an Rosen,
- Elsinoe fawcetti und Diaporthe citri an Citrus-Früchten,
- Sphaerotheca-Arten an Kürbisgewächsen, Erdbeeren und Rosen,
- Cercospora-Arten an Erdnüssen, Zuckerrüben und Auberginen,
- Erysiphe cichoracearum an Kürbisgewächsen,
- Leveillula taurica an Paprika, Tomaten und Auberginen,
- Mycosphaerella-Arten an Äpfeln und japanischer Aprikose,
- Phyllactinia kakicola, Gloesporium kaki, an japanischer Aprikose,
- Gymnosporangium yamadae, Leptothyrium pomi, Podosphaera leucotricha und Gloedes pomigena an Äpfeln,
- Cladosporium carpophilum an Birnen und japanischer Aprikose,
- Phomopsis-Arten an Birnen,
- Phytophthora-Arten an Citrusfrüchten, Kartoffeln, Zwiebeln, insbesondere Phytophthora infestans an Kartoffeln und Tomaten,
- Blumeria graminis (echter Mehltau) an Getreide,
- Fusarium- und Verticillium-Arten an verschiedenen Pflanzen,
- Glomerella cingulata an Tee,
- Drechslera- und Bipolaris- Arten an Getreide und Reis,
- Mycosphaerella-Arten an Bananen und Erdnüssen,
- Plasmopara viticola an Reben,
- Personospora-Arten an Zwiebeln, Spinat und Chrysantemen,
- Phaeoisariopsis vitis und Sphaceloma ampelina an Grapefruits,
- Pseudocercosporella herpotrichoides an Weizen und Gerste,
- Pseudoperonospora-Arten an Hopfen und Gurken,
- Puccinia-Arten und Typhula-Arten an Getreide und Rasen,
- Pyricularia oryzae an Reis,
- Rhizoctonia-Arten an Baumwolle, Reis und Rasen,
- Stagonospora nodorum und Septoria tritici an Weizen,
- Uncinula necator an Reben,
- Ustilago-Arten an Getreide und Zuckerrohr, sowie
- Venturia-Arten (Schorf) an Äpfeln und Birnen.

Die Verbindungen I eignen sich außerdem zur Bekämpfung von Schadpilzen wie Paecilomyces variotii im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittle-. rem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt.
   Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zinkethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylenbis-(thiocarbamoyl)disulfid;
- Nitroderivate, wie Dinitro-(1-methylheptyl)-phenylcrotonat, 7-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
- heterocyclische Substanzen, wie 2-Heptadecyl-2-imidazolin-acetat, 2-Chlor-N-(4'-chlor-biphenyl-2-yl)-nicotinamid, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4-triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
- N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methylfuran-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-ylethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
- Strobilurine wie Methyl-E-methoxyimino-[α-(o-tolyloxy)-o-tolyl]acetat, Methyl-E-2-{2-[6-(2-cyanophenoxy)-pyrimidin-4-yloxy]-phenyl}-3-methoxyacrylat, Methyl-E-methoxyimino-[α-(2-phenoxyphenyl)]-acetamid, Methyl-E-methoxyimino-[α-(2,5-dimethylphenoxy)-o-tolyl]-acetamid, Methyl-E-2-{2-[2-trifluormethylpyridyl-6-]oxymethyl]-phenyl}3-methoxyacrylat, (E,E)-Methoximino-{2-[1-(3-trifluormethylphenyl)-ethylidenaminooxymethyl]-phenyl}-essigsäuremethylester, Methyl-N-(2-{[1-(4-chlorphenyl)-1H-pyrazol-3-yl]oxymethyl}phenyl)N-methoxy-carbamat,
- Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin,
- Phenylpyrrole wie 4-(2,2-Difluor-1,3-benzodioxol-4-yl)-pyrrol-3-carbonitril,
- Zimtsäureamide wie 3-(4-Chlorphenyl)-3-(3,4-dimethoxyphenyl)-acrylsäuremorpholid, 3-(4-Fluorphenyl)-3-(3,4-dimethoxyphenyl)-acrylsäuremorpholid,
- sowie verschiedene Fungizide, wie Dodecylguanidinacetat, 1-(3-Brom-6-methoxy-2-methyl-phenyl)-1-(2,3,4-trimethoxy-6-methyl-phenyl)-methanon, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-(3,5-Dichlorphenyl)-5-methyl-5-methoxymethyl-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1 H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol, 5-Chlor-2-cyano-4-p-tolyl-imidazol-1-sulfonsäuredimethylamid, 3,5-Dichlor-N-(3-chlor-1-ethyl-1-methyl-2-oxo-propyl)-4-methylbenzamid.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

### Beispiel 1: Herstellung von 3,4,8-Trimethoxy-1-methyl-xanthen-9-on [I-1]:

### Beispiel 1a: 1-(2,6-Dichlor-phenyl)-1-(2-hydroxy-3,4-dimethoxy-6-methyl-phenyl)-methanon

Eine Lösung von 40 g (0,3 mol) Aluminiumtrichlorid in 400 ml Nitrobenzol, wurde mit einer Lösung von 52,4 g (0.25 mol) o,o'-Dichlorbenzoylchlorid in Nitrobenzol bei 20-25 °C versetzt. Nach 30 min Rühren wurden 45,5 g (0,25 mol) in Nitrobenzol gelöstes Trimethoxytoluol zugetropft. Nach etwa zwei Stunden Rühren bei 50°C wurde Wasser zugegeben, dann zweimal mit Dichlormethan extrahiert. Die vereinten organischen Phasen wurden getrocknet und vom Lösungsmittel befreit. Nach Chromatographie an Kieselgel (Essigsäureethylester/Cyclohexan-Gemische) wurden 40,0 g der Titelverbindung erhalten.

### Beispiel 1b: Cyclisierung zu 3,4,8-Trimethoxy-1-methyl-xanthen-9-on [I-1]

Eine Lösung von 2,6 g Natrium (110 mmol) in 50 ml Methanol wurde unter Schutzgasatmosphäre bei etwa 0-5°C mit 37,5 g (0,11 mol) des Benzophenonderivates aus Beispiel 1a in 100 ml Dimethoxyethan versetzt. Nach etwa 72 Stunden Rühren bei 80°C wurde die Titelverbindung durch Zugabe eines 1:1-Gemisches aus Wasser und Essigsäureethylester ausgefällt. Durch Filtration wurden 33,4 g (100% d. Th.) vom Fp. 158°C isoliert.

### Beispiel 2: Herstellung von 5-Brom-3,4,8-trimethoxy-1,8-dimethylxanthen-9-on [I-2]:

Eine Lösung von 33,4 g des Xanthons aus Beispiel 1b in 100 ml Dichlormethan wurde mit 9,4 g (5,9 mmol) Brom bei etwa 0°C versetzt. Nach etwa 16 Stunden Rühren bei 20-25°C wurde Eiswasser zugegeben und mit Dichlormethan extrahiert. Die vereinten organischen Phasen wurden mit Wasser sowie mit ges. Natriumhydrogencarbonatlösung gewaschen und getrocknet. Durch Entfernen des Lösungsmittels wurden 2,1 g (5,5 mmol, 93% d. Th.) der Titelverbindung erhalten.

### Beispiel 3: Herstellung von 7-Chlor-3,4-dimethoxy-1,8-dimethylxanthen-9-on [I-5]

### Beispiel 3a: 4-Chlor-5-methyl-salicylsäuremethylester

Zu einer Lösung von 11 g (66 mmol) o-Methylsalicylsäuremethylester in 600 ml Eisessig wurden bei 80°C 9,9 g (73 mmol) Sulfurylchlorid zugetropft. Die Lösung wurde bei 100°C etwa acht Stunden und bei etwa 20 bis 25°C weitere 12 Std. gerührt. Die Reaktionsmischung wurde auf Wasser gegossen, dann mit Essigsäureethylester extrahiert. Die vereinten organischen Phasen wurden mit Wasser gewaschen, getrocknet und vom Lösungsmittel befreit. Es wurden 12,85g (64 mmol) der Titelverbindung (97% d. Th.) erhalten und in der Folgestufe umgesetzt.

### Beispiel 3b: 4-Chlor-5-methyl-salicylsäure

Eine Lösung von 12,85 g (64 mmol) des Esters aus Bsp. 3a in 80 ml Wasser und 130 ml Ethanol wurde nach Versetzen mit 5,12 g (128 mmol) NaOH etwa 15 Std. refluxiert. Die Reaktionsmischung wurde auf Wasser gegossen und mit Essigsäureethylester gewaschen. Nach Phasentrennung wurde die wässrige Phase angesäuert und mit Essigsäureethylester extrahiert. Die vereinten organischen Phasen wurden mit Wasser gewaschen, getrocknet und vom Lösungsmittel befreit. Es wurden 10,4 g (55,8 mmol, 87% d. Th.) der Titelverbindung als gelbliche Kristallmasse erhalten.

### Beispiel 3c: 3-Chlor-6-(2,2-dimethyl-propanoyloxy)-2-methyl-benzoesäure

Einer Lösung von 2,35 g (12,6 mmol) der Säure aus Bsp. 3b in 30 ml Tetrahydrofuran wurden 1,2 ml Pyridin und 1,62 g (13,48 mmol) Pivaloylchlorid zugesetzt. Nach etwa 15 Std. Rühren bei etwa 20 bis 25°C wurde die Reaktionsmischung auf Wasser gegossen, dann mit Essigsäureethylester extrahiert. Die vereinten organischen Phasen wurden getrocknet und vom Lösungsmittel befreit. Aus dem Rückstand wurden 3,45 g (100 % d. Th.) der Titelverbindung erhalten.

### Beispiel 3d: 2,2-Dimethyl-propionsäure-4-chlor-3-methyl-2-[1-(2,3,4-trimethoxy-6-methyl-phenyl)-methanoyl]-phenylester

Zu einer Lösung von 3,45 g (12,6 mmol) der Benzoesäure aus Bsp. 3c in 50 ml Dichlormethan (CH₂Cl₂) wurden 2,3 g (12.6 mmol) Trimethoxytoluol und 4 g pulverisiertes P₂O₅ gegeben. Nach etwa 15 Std. Rühren bei etwa 20 bis 25°C wurde die Reaktionsmischung auf Wasser gegossen, dann mit CH₂Cl₂ extrahiert. Die vereinten organischen Phasen wurden mit Wasser gewaschen, getrocknet und vom Lösungsmittel befreit. Aus dem Rückstand wurden 4,9 g (89% d. Th.) der Titelverbindung erhalten.

### Beispiel 3e: 7-Chlor-3,4-dimethoxy-1,8-dimethyl-xanthen-9-on [I-5]

Eine Lösung von 22 g ( 50.6 mmol) des Pivaloylderivates aus Bsp. 3d in 400 ml Methanol und 160 ml Wasser wurde nach Versetzen mit 15,7 g (148 mmol) Natriumhydrogencarbonat etwa eine Stunde refluxiert und bei etwa 20 bis 25°C weitere 15 Std. gerührt. Die Reaktionsmischung wurde auf Wasser gegossen und mit Essigsäureethylester extrahiert. Die vereinten organischen Phasen wurden mit Wasser gewaschen, getrocknet und vom Lösungsmittel befreit. Der Rückstand wurde einer Chromatographie an Kieselgel (Methyltert-butylether/Hexan 1:20) unterworfen. Die Produktfraktion wurde vom Lösungsmittel befreit und in Petrolether digeriert. Es wurden 0,41 g der Titelverbindung vom Fp. 101°C erhalten.

### Beispiel 4: Alternative Herstellung von 7-Chlor-3,4-dimethoxy-1,8-dimethyl-xanthen-9-on [I-5]

### Beipiel 4a: 1-(3-Chlor-6-hydroxy-2-methyl-phenyl)-1-(2,3,4-trimethoxy-6-methyl-phenyl)-methanon

Eine Lösung von 2,8 g (6,44 mmol) der Verbindung aus Bsp. 3d in 60 ml Methanol und 20 ml Wasser wurde nach Versetzen mit 2 g (21 mmol) NaHCO₃ fünf Stunden refluxiert und für 15 Std. bei etwa 20-25°C gerührt. Nach Gießen auf Wasser wurde mit Essigsäureethylester extrahiert. Die vereinten organischen Phasen wurden mit Wasser, dann mit ges. NaHCO₃-Lösung gewaschen, getrocknet und und vom Lösungsmittel befreit. Nach Digerieren in Petrolether wurde der Rückstand abfiltriert. Es wurden 1,7 g (75% d. Th.) der Titelverbindung erhalten.

### Beispiel 4b: 7-Chlor-3,4-dimethoxy-1,8-dimethyl-xanthen-9-on [I-5]

Eine Lösung von 1,5 g (4,3 mmol) des Produkts aus Bsp. 4a in 15 ml N-Methylpyrrolidon und 30 ml Methanol wurde mit 1,2 g (21,5 mmol) KOH versetzt, dann für fünf Std. refluxiert. Nach Abkühlen wurde die Reaktionsmischung auf Wasser gegeben, dann mit Dichlormethan extrahiert. Die vereinten organischen Phasen wurden mit Wasser gewaschen und getrocknet. Nach dem Entfernen des Lösungsmittels und chromatographischer Reinigung des Rückstandes (Methyl-tert.-butylether:Hexan 1:9) wurden 0,72 g der Titelverbindung als farblose Kristalle vom Fp. 101°C erhalten.

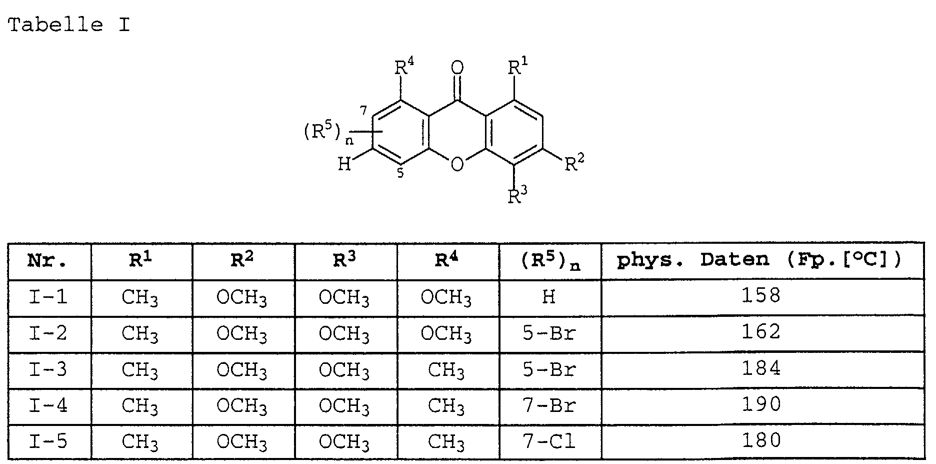

### Beispiele für die Wirkung gegen Schadpilze

Die fungizide Wirkung der Verbindungen der Formel I ließ sich durch die folgenden Versuche zeigen:

Die Wirkstoffe wurden getrennt oder gemeinsam als 10%ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl) aufbereitet und entsprechend der gewünschten Konzentration mit Wasser verdünnt.

### Anwendungsbeispiel - Wirksamkeit gegen Weizenmehltau

Blätter von in Töpfen gewachsenen Weizenkeimlingen der Sorte "Kanzler" wurden mit wäßriger Wirkstoffaufbereitung, die aus einer Stammlösung bestehend aus 10 % Wirkstoff, 63 % Cyclohexanon und 27 % Emulgiermittel angesetzt wurde, bis zur Tropfnässe besprüht und 24 Stunden nach dem Antrocknen des Spritzbelages mit Sporen des Weizenmehltaus (Blumeria graminis forma specialis tritici) bestäubt. Die Versuchspflanzen wurden anschließend im Gewächshaus bei Temperaturen zwischen 20 und 24°C und 60 bis 90 % relativer Luftfeuchtigkeit aufgestellt. Nach 7 Tagen wurde das Ausmaß der Mehltauentwicklung visuell in % Befall der gesamten Blattfläche ermittelt.

In diesem Test zeigten die mit 4 und 16 ppm der Verbindung I-5 behandelten Pflanzen nicht über 3% Befall, während die unbehandelten Pflanzen zu 90 % befallen waren.

## Patentansprüche

1. Xanthonderivate der Formel I 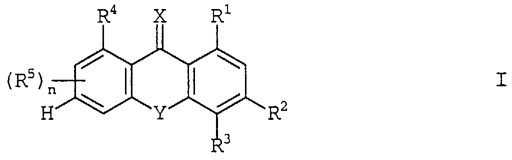 in der der Index und die Variablen folgende Bedeutung haben
n 0, 1 oder 2;
R¹ C₁-C₄-Alkyl oder C₁-C₄-Halogenalkyl;
R²,R³ unabhängig voneinander Wasserstoff, C₁-C₆-Alkoxy, C₃-C₆-Alkenyloxy oder C₃-C₆-Alkinyloxy,
oder R² und R³ bilden gemeinsam eine Oxy-C₁-C₄-Alkylenoxygruppe, die unsubstituiert oder durch 1 bis 4 der folgenden Reste substituiert ist: Halogen, Cyano, Hydroxy oder C₁-C₄-Alkyl;
R⁴ Halogen, Cyano, Hydroxy, Amino, Mercapto, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylcarbonyloxy oder C₁-C₄-Alkylcarbonylthio;
R⁵ eine Gruppe R⁴, wobei die Gruppen R⁵ verschieden sein können, wenn n=2 ist;
X, Y unabhängig voneinander Sauerstoff oder Schwefel.

2. Verbindungen der Formel I gemäß Anspruch 1, in der X und Y Sauerstoff bedeuten.

3. Verbindungen der Formel I gemäß Ansprüchen 1 oder 2, in der R¹ Methyl bedeutet.

4. Verbindungen der Formel I gemäß Ansprüchen 1 bis 3, in der R² und R³ Methoxy, Ethoxy, n- oder iso-Propoxy oder n-Butoxy bedeuten.

5. Verbindungen der Formel I gemäß Ansprüchen 1 bis 4, in der R⁴ Methyl und R⁵ Halogen bedeuten.

6. Verfahren zur Herstellung von Verbindungen der Formel I gemäß Anspruch 1, in der X Sauerstoff bedeutet, durch Kondensation von Alkylbenzolderivaten der Formel II 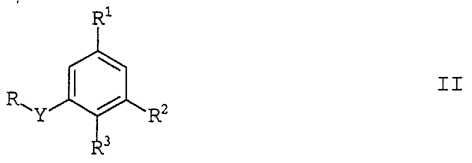 in der R für eine C₁-C₄-Alkylgruppe steht und die anderen Variablen die Bedeutung wie in Formel I haben, mit Salicylsäurederivaten der Formel III, 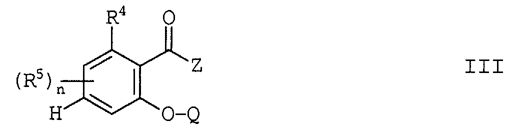 in der Z Hydroxy, Halogen oder C₁-C₄-Alkoxy, Q eine Schutzgruppe bedeutet und R⁴ und (R⁵)ₙ die Bedeutung wie in Formel I haben, zu Verbindungen IV, 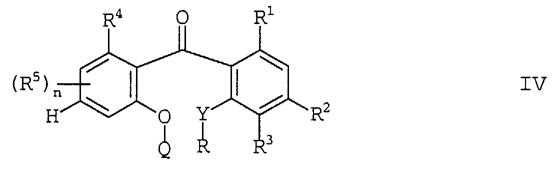 abspaltung von Q und Cyclisierung zu den Verbindungen der Formel I.

7. Verfahren zur Herstellung von Verbindungen der Formel I gemäß Anspruch 1, in der X und Y Sauerstoff und R⁴ Hydroxy, C₁-C₄-Alkoxy oder C₁-C₄-Halogenalkoxy bedeuten, durch Kondensation von Alkylbenzolderivaten der Formel II.1, 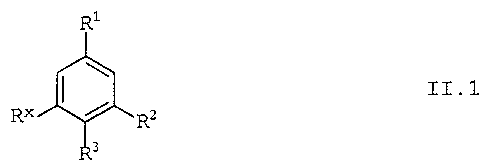 in der R^{x} für C₁-C₃-Alkoxy steht, und Benzoesäurehalogeniden der Formel III.1 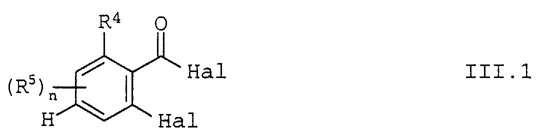 in der Hal für Halogen steht, zu Verbindungen der Formel IV.1 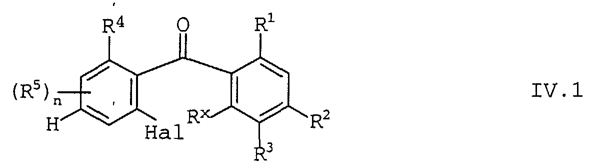 und basenkatalysierte Cyclisierung von IV.1 in Gegenwart Basen zu Verbindungen der Formel I.

8. Verwendung der Verbindungen I gemäß Ansprüchen 1 bis 5 zur Bekämpfung von pflanzenpathogenen Schadpilzen.

9. Zur Bekämpfung von Schadpilzen geeignetes Mittel, enthaltend einen festen oder flüssigen Trägerstoff und eine Verbindung der allgemeinen Formel I gemäß Anspruch 1.

10. Verfahren zur Bekämpfung von pflanzenpathogenen Schadpilzen, **dadurch gekennzeichnet, daß** man die Pilze oder die vor Pilzbefall zu schützenden Materialien, Pflanzen, den Boden oder Saatgüter mit einer wirksamen Menge einer Verbindung der allgemeinen Formel I gemäß Anspruch 1 behandelt.
